# EUROPEAN PATENT SPECIFICATION

(11) **EP 2 266 522 B1**
(45) Date of publication and mention of the grant of the patent: **17.10.2012**
(21) Application number: 10009808.6
(22) Date of filing: 11.04.1997
(51) Int. Cl.: A61J 1/10, B65B 55/16

(54) **Carrier and method for containers to be sterilized by E-beams**
Träger und Verfahren für das Sterilisieren von Behältern mit E-Strahlen
Support et méthode pour la stérilisation de conteneurs par faisceaux d'électrons

(30) Priority: 13.05.1996 US 647583
(43) Date of publication of application: 29.12.2010
(62) Divisional of application: 05014220.7
(73) Proprietor: B. Braun Medical, Inc., Bethlehem PA 18018-0027 (US)
(72) Inventor: Barney, Ward W., Bethlehem, PA 18018-0027 (US); McLonis, Mark R., Bethlehem, PA 18018-0027 (US); Sacca, Giuseppe, Bethlehem, PA 18018-0027 (US); Smith, Steven L., Bethlehem, PA 18018-0027 (US); York, Walter A., Bethlehem, PA 18018-0027 (US); Gharibian, Noel, Bethlehem, PA 18018-0027 (US); Pearson, Christopher D., Bethlehem, PA 18018-0027 (US); Sandberg, Sharilyn J., Bethlehem, PA 18018-0027 (US); Walter, William V., Bethlehem, PA 18018-0027 (US); Young, H. Theodore, Bethlehem, PA 18018-0027 (US); Havey, Douglas G., Bethlehem, PA 18018-0027 (US); Pool, Scott L., Bethlehem, PA 18018-0027 (US); Sakaguchi, Thomas R., Bethlehem, PA 18018-0027 (US); Wu, Nicholas Chung-Hui, Bethlehem, PA 18018-0027 (US)
(74) Representative: Grünecker, Kinkeldey, Stockmair & Schwanhäusser

(56) References cited:
- WO-A1-94/22162
- JP-A- 06 205 818
- US-A- 4 481 652
- US-A- 5 496 302

## Description

The present invention relates to a method for sterilizing a flexible storage container comprising a flexible rear sheet and a flexible front sheet according to claim 10 and to a transport carrier for sterilizing flexible storage container according to claim 1.

### SUMMARY OF THE INVENTION

The present invention provides a container having multiple compartments separated by peelable seals which may be ruptured by manually applying pressure to the exterior of the container. The container is formed of two sheets of flexible, laminated materials which are sealed together along their perimeters. Separate compartments in the container are formed by peelable heat seals.

In an additional embodiment of the invention, unfilled container are placed in a transport carrier which is then sealed against environmental contamination. The transport carrier, and the containers within, are subject to E-beam sterilization. The transport carrier, and containers within, are introduced into the isolator through a UV decontamination tunnel, which ensures maintenance of a sterile environment inside the isolator.

### BRIEF DESCRIPTION OF THE DRAWINGS

These and other features, aspects and advantages of the present invention will be more fully understood when considered with regard to the following detailed description, appended claims and accompanying drawings wherein:
FIG. 1 is a semi-schematic front view of an exemplary embodiment of a container provided in accordance with the present invention at an intermediate stage of its fabrication showing the arrangement of sacrificial ports for filling of the container;
FIG. 1a is a semi-schematic side view of an exemplary embodiment of a container, detailing the arrangement and construction of the powder and liquid fill sacrificial ports including a cap in accordance with the present invention;
FIG. 1b is a semi-schematic top view of the sacrificial ports of FIG. 1a detailing the shape and arrangement of the port flanges;

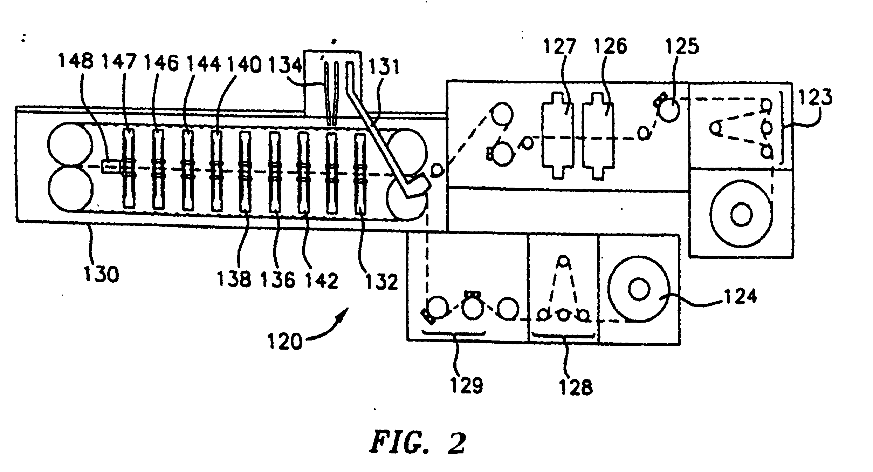

FIG. 2 is a semi-schematic plan view of an embodiment of a modular container fabrication apparatus in accordance with the present invention;
FIG. 3 is a semi-schematic perspective view of a handling container provided in accordance with the present invention, including a container tray for receiving a rail cartridge and covered by a scalable film lid;
FIG. 4a is a semi-schematic perspective view of the components of the rail cartridge of FIG. 10, depicting the rail cartridge in exploded form and ready for assembly;
FIG. 4b is semi-schematic perspective view of the completely assembled rail cartridge of FIG. 4a;
FIG. 5a is a semi-schematic plan view of the rail cartridge of FIGS. 4a and 4b depicting containers loaded onto the rails in accordance with the present invention;
FIG. 5b is a semi-schematic front view of the loaded rail cartridge of FIG. 5a showing how containers are held within the rails by the sacrificial ports;
FIG. 6 is a flow chart of the sterilization and aseptic filling process for one embodiment of a container in accordance with one embodiment of the present invention;
FIG. 7 is a semi-schematic plan view of an embodiment of a modular container filling apparatus showing the progressive process stations in accordance with practice of the present invention;
FIG. 8a is a semi-schematic pictorial cut-away view of a portion of a container transport band in accordance with practice of the present invention;
FIG. 8b is a semi-schematic partial perspective view of the arrangement of a powder filling wheel and transport band showing the direction of travel of containers beneath the filling wheel.

### DETAILED DESCRIPTION

In order for an aseptic filling process to be acceptable for medical purposes, the container to be filled must be provided in a sterile condition. Conventionally, container sterilization takes place in a separate processing area or facility due to the rather extensive and complex equipment and processes required for sterilizing material. A particular undesirable feature of the sterilization procedure is that the container must be transported to the sterilization facility for processing, following which container sterility must be maintained during subsequent storage and transport to an aseptic filling facility. The container must be introduced into the aseptic filling zone by means of a sterile transfer in order to prevent contamination of the aseptic zone by the container. Once introduced into the aseptic zone, the container may be filled aseptically, but must be further handled in a sterile fashion.

In accordance with practice of principles of the invention, following primary container fabrication, a plurality of empty containers are loaded into a handling containers which is then sealed to protect the bags contained within from environmental contamination.

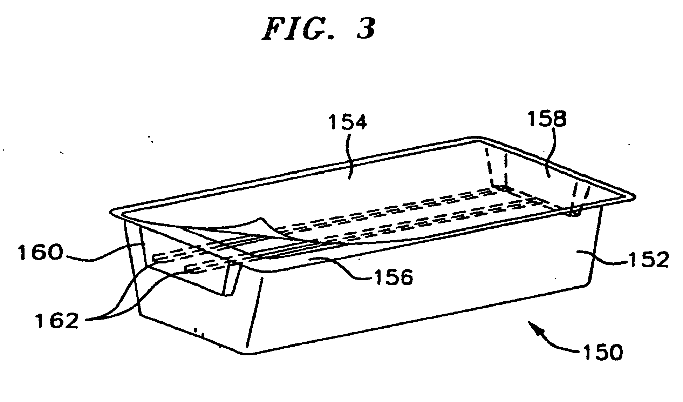

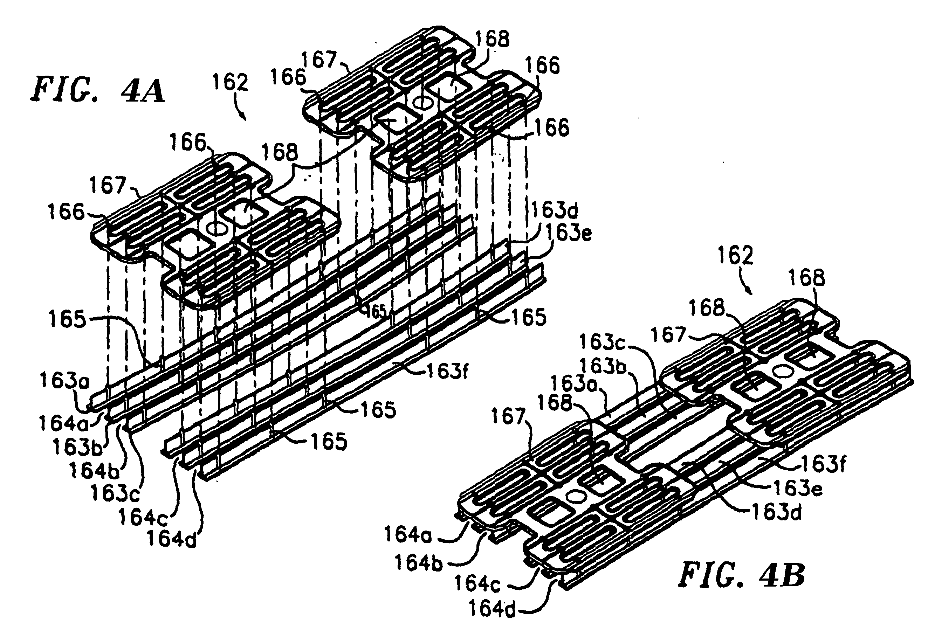

Turning now to FIG. 3, a handing container, generally indicated at 150 and termed "a carrier" herein, functions as a transportable sterile containment isolator for sterilizing, transporting and introducing into the aseptic zone, empty containers in a systematic manner. The carrier 150 comprises three components a generally rectangular container tray 152, a sealable film lid 154, and a rail cartridge 162 for supporting a multiplicity of containers within the tray and which will be described in greater detail below in connection with FIGs. 4a & 4b.

The generally rectangular container tray 152 is constructed of a thermoformed polystyrene material chosen to be able to withstand several sterilization cycles without significant degradation. The tray 152 is shaped generally in the form of a basin with its upper peripheral edge bent-over outwardly to form a flat, horizontally oriented peripheral lip 156 which extends beyond the sides of the tray 152 for a distance of between about 1/4 inches to about 1 inch. Preferably, the lip 156 extends about 3/4 inches beyond the sides of the tray, but any extension which provides rigidity to the tray 152 and a sufficient surface to support a seal is suitable. Two opposing pockets 158 and 160 are formed in about the centers of the two opposing short sides of the tray and extend outward from the plane of the short sides. The pockets 158 and 160 extend only partially downward along the sides of the tray and form, thereby, two opposing recesses into which the ends of the rail cartridge 162 may be inserted. The rail cartridge 162 rests on the bottom surfaces of the pockets 158 and 160 and is thereby suspended above the bottom of the tray 152 at a height sufficient to allow containers arranged on the rail cartridge to hang free within the interior volume of the tray. Accordingly, the pockets 158 and 160, in combination with the rail cartridge 162, functions to maintain a multiplicity of containers in a specific orientation during transport, storage and UV sterilization.

Once the rail cartridge 162 has been loaded with containers and inserted into the pockets 158 and 160, the tray 152 is environmentally sealed by heat sealing the plastic film lid 154 to the tray flange 156 in a distinct orientation. For illustrative purposes in FIG. 3, the film lid 154 is depicted half way through the sealing process, with a portion of the lid lifted up to show the rail cartridge 162 nested within the tray 152. The film lid 154 is positioned on the flange 156 such that there is no "overhang" of the film lid material over the edge of the tray flange around the perimeter of the tray. In an exemplary embodiment, the plastic film lid 154 is constructed to have dimensions which allow the film lid to be positioned on the tray flange such that the film lid edge is inset from the tray flange edge around the entire flange periphery. In addition, the film lid heat seal is applied to extend beyond the edge of the film lid 154, to assure that no portion of the film lid edge left unsealed that would create a loose edge "flap". Film lid orientation, placement and the avoidance of loose edges is particularty important to the surface ultraviolet (UV) decontamination process performed on the carrier 150 when the carrier is introduced to the aseptic zone. Crevices, caused by loose firm lid edges and/or flaps, may cause a local shadow, when exposed to UV radiation, which shadowing effect can defeat the UV decontamination process.

Once the film lid 154 has been heat sealed to the tray flange 156, the carrier 150 defines a hermetically sealed environment that functions to isolate its contents from external contamination. The carrier 150 is subsequently placed into a polybag overwrap (not shown), which acts a "dust cover", and identified with an adhesive label which is placed on the over wrap.

Turning now to FIGs. 4a and 4b, the carrier rail cartridge 162 is depicted in its component form, ready for assembly, in FIG. 4a and in a fully assembled condition in FIG. 4b. The carrier rail cartridge 162 suitably comprises a plurality of injection molded, polystyrene T-beams 163a, b, c, d, e, and f, disposed at spaced-apart intervals so as to form longitudinally running slots 164 a, b, c, and d therebetween. The polystyrene T-bars 163a-f are oriented with the legs of the T facing upwards (from the perspective of FIGs. 4a and 4b) and include press or snap-fit pins 165, adapted to mate with corresponding receptacles 166 on one or more spacer plates 167. The spacer plates 167, like the T-rails 163a-f, are constructed of an injection molded high impact polystyrene material such as FINA 825 manufactured and sold by Fina Oil and Chemical Company of Deerpark Texas. The spacer plates 167 of the carrier rail cartridge 162 are provided to separate and maintain the T-rails 163a-f at pre-determined distances from one another. The spacer plates may include cutouts 168 arranged to provide hand holds so that the final carrier rail cartridge assembly may be easily grasped, lifted and moved. Alternatively, a thin, flexible plastic handle may be attached to span spacer plates 167, or some other well known means may be provided by which the carrier rail cartridge may be grasped and manipulated

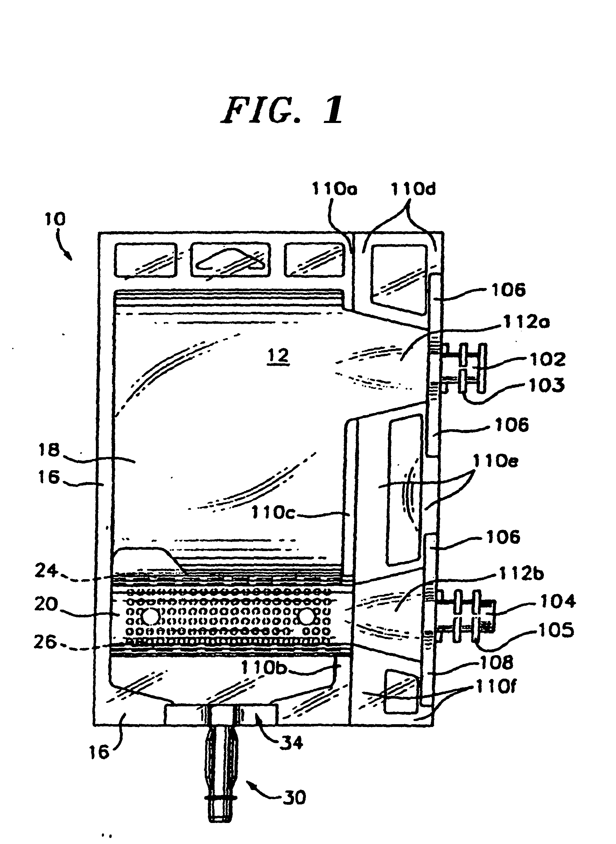

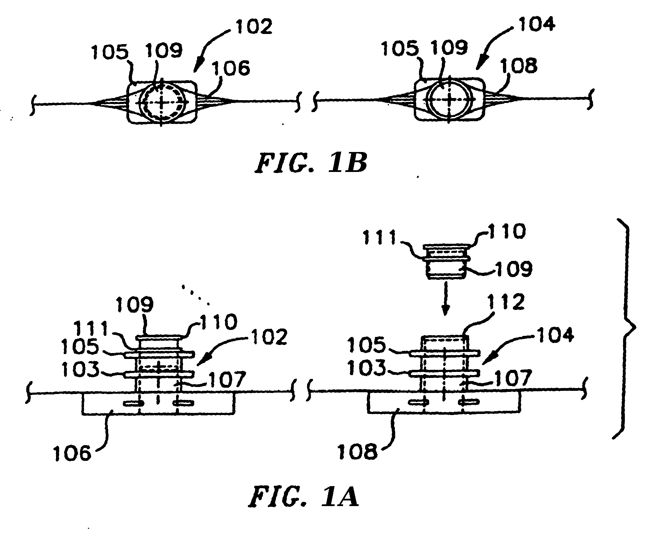

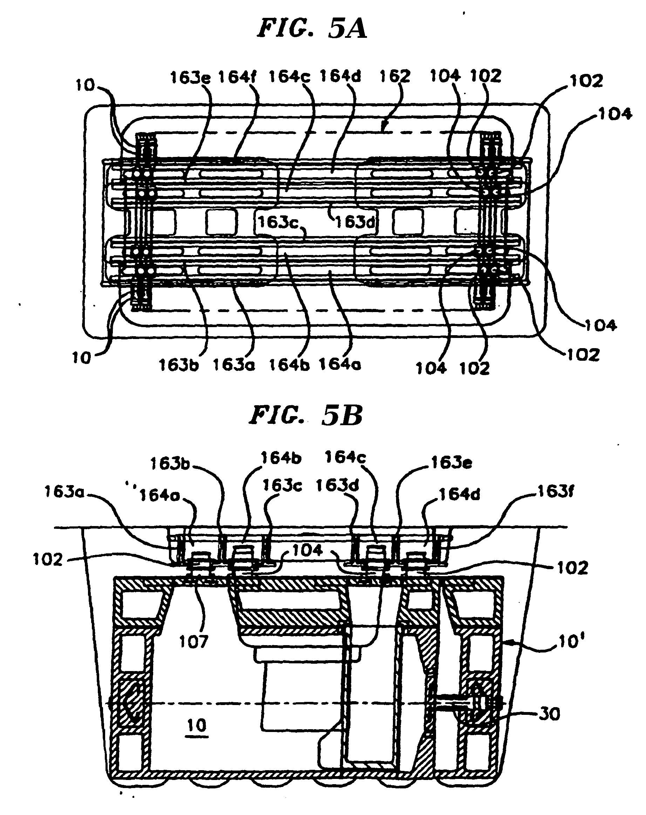

Once the carrier rail cartridge has been assemble fabricated containers may be loaded onto the cartridge in accordance with the invention, in a manner depicted in FIGs. 5a and 5b. In FIG. 5a, which is a plan view of a loaded carrier rail cartridge, finished containers 10, such as those depicted in FIG. 1, are loaded onto the carrier rail cartridge 162 by inserting their sacrificial ports (102, 104) into the slots 164a-d formed between the cartridge's T-rails 163a-f, in the manner depicted in FIG. 5b. The flange edges of the T-rails 163a-f are spaced-apart a sufficient distance (about 13.0 mm) such that the central filling barrel 107 of each sacrificial port is able to be accommodated therebetween, and are adapted to engage the sacrificial ports between the port's circumferential flanges (103 and 105 of FIG. 1) such that each container 10 is grasped by the T-rail flanges beneath its uppermost circumferential sacrificial port flange 105.

In the exemplary embodiment of the carrier rail cartridge depicted in FIGs. 5a and 5b, four slots 164a, b, c, and d are provided for receiving containers, with the containers loaded onto the rail cartridge 162 in alternating left and right orientations. The sacrificial ports 102 and 104 of each container are inserted into two of the slots 164a-f. As depicted in FIG. 5b, a first container 10' is loaded into the second and fourth slots (164b and 164d) and is oriented in a first horizontal direction, such that its hanger end is oriented to the right, (from the perspective of FIG. 5b), and its set port is oriented to the left. The second container 10 (the front container from the perspective of FIG. 5b) is loaded onto the rail cartridge 162 with its sacrificial ports 102 and 104 inserted into the first and third cartridge slots 164a and 164c. The second container 10 is loaded in a second horizontal direction with its set port 30 oriented 180° with respect to the first container. In the example of FIG. 5b, the set port 30 of the first container 10 is on the right hand side when viewed from the perspective of FIG. 5b. Further containers are loaded onto the carrier rail cartridge 162 in like fashion, with the container's horizontal orientation alternating left and right; the sacrificial ports of the left oriented containers inserted into the second and fourth slots, the set ports of the right oriented containers loaded into the first and third slots, as described above, until the carrier rail cartridge 162 is completely filled.

Returning now to FIC3. 5a, it will be understood that the particular design of the flanges of the sacrificial ports 102 and 104, in cooperation with the carrier rail cartridge 162, functions to maximize the packing density of containers within the container tray 152. As can be seen in FIG. 5a, the sacrificial port flanges protrude only along the length direction of each container, and not along its width or thickness dimension. Accordingly, as the carrier rail cartridge is filled, the thickness of any one particular container is defined by the width of its sacrificial port barrel (approximately 12.0 mm). As succeeding containers are loaded onto the carrier rail cartridge, only the filling barrels of the sacrificial ports of alternating containers come into contact with one another. Alternating the horizontal orientation of consecutive containers, as well as alternating their slot offset position, also assists in enhancing the packing density of containers in a fully loaded rail cartridge. As can be seen from FIG. 5a, providing a second set of slots allows the container packing density to be substantially doubled, in contrast to a carrier rail cartridge system with only a single pair of slots.

It will be evident to one having skill in the art that containers are loaded onto the carrier rail cartridge and held therein in a systematically aligned orientation such that each alternating container is 180° opposed to the previous container as well as being laterally offset from the previous container by the slot spacing of the cartridge. It will be understood that this alternating container orientation maximizes the parking density of containers along the length of the cartridge as well as defining specific orientations and locations of a multiplicity of containers with respect to the cartridge rails, for easy adaptation of the cartridge assembly to an automated loading and unloading system. The carrier rail cartridge and container loading sequence allows the cartridge to be temporarily "docked" or mounted to pick-and-place robotic machinery. Further, the cutouts (or thumb holes) 168 in spacing plates 167, allow an operator to easily insert and remove a fully loaded cartridge from the carrier tray without unduly tilting the cartridge, thus minimizing the possibility of containers falling off the rail.

Following loading, the carrier rail cartridge is placed within the tray 152 with the ends of the T-rails 163a-f nested in the pockets 158 and 160 formed in the ends of the tray. Pockets 158 and 160 support the carrier rail cartridge 162 within the interior volume of the tray and provide additional lateral support which prevents the cartridge from shifting during shipping, sterilization and storage.

The sealed carrier, including the empty containers within, is wrapped in a poly bag and transported to a radiation sterilization facility where it and its contents are rendered sterile by an E-beam sterilization procedure, for example

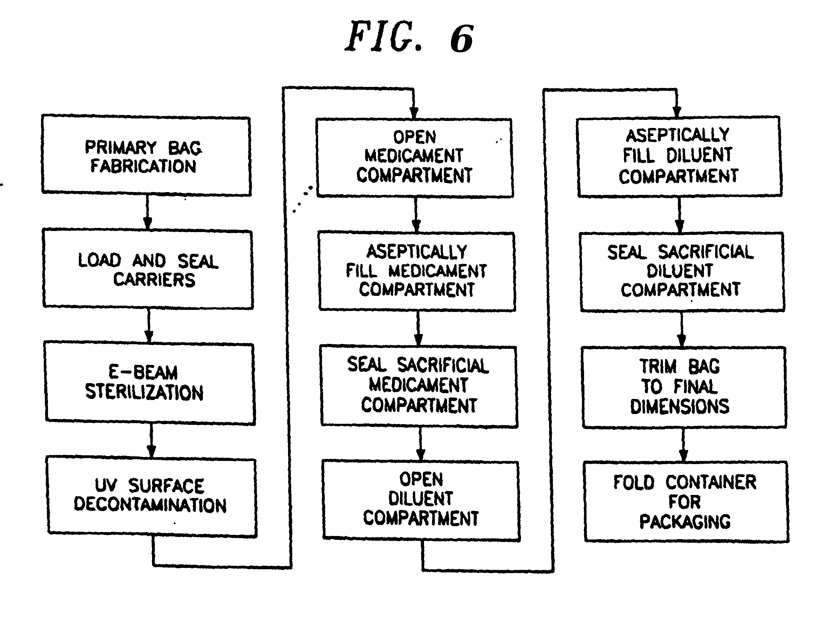

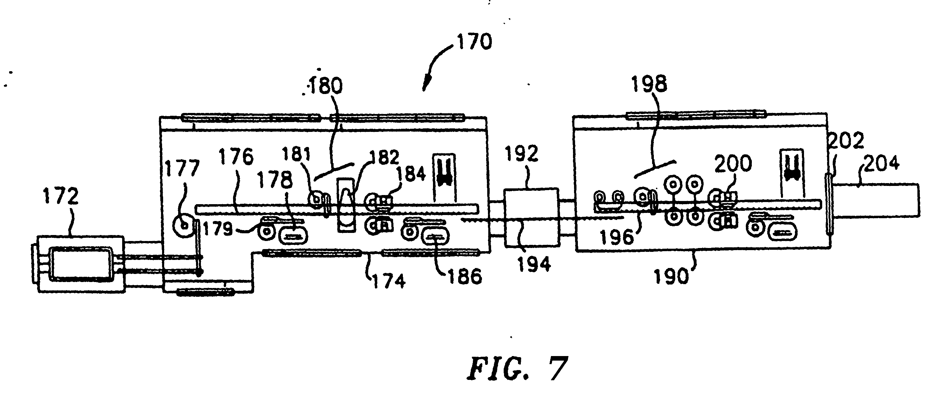

After the foregoing carrier loading and E-beam sterilization procedure is completed, the sterilized medical containers are transported to an aseptic filling facility, and the containers are aseptically filled in accordance with one embodiment of the invention as is described with reference to an exemplary process flow-chart depicted in FIG. 6 and an exemplary filling apparatus depicted in semi-schematic, plan view in FIG. 7.

Primary bag filling will take advantage of manufacturing technology developed in connection with integrated circuit fabrication that is becoming more common in the medical industry. This technology generally involves a move away from conventional container filling in class 100 aseptic environments, to container filling within an "isolator" unit in which the environment is sterile. The main distinction between class 100 aseptic environments and "isolators" is the separation of the worker from the environment. An isolator is in essence, a "mini environment" which encloses the immediate machinery and container filling operation within a controlled space. The worker is separated from this space and interfaces with the materials therein through glove ports and/or "half suits". By separating the worker from the environment, it is possible to create and maintain a small, sterile environment, since the worker is typically the major source of biological contaminates.

The isolator is initially sterilized with a sterilant such as vaporized hydrogen peroxide (VHP). Inside the isolator, the ambient atmosphere is maintained in the sterile condition by supplying it with HEPA and ULPA filtered air. The ambient atmosphere within the isolator is also maintained at a higher pressure than the ambient atmosphere surrounding the isolator. Positive air pressure ensures that air flow is always from the inside of the isolator to the outside. All components or sub-assemblies that will enter the isolator are either pre-sterilized or sterilized just prior to placing them in the isolator so that the sterile environment is maintained. Components are typically placed in isolators via doors or through entry/exit ports commonly referred to as RTPs (rapid transfer ports). RTPs are designed to mechanically interlock in a manner such that sterility is not compromised. Containers which cany sterile components to the isolator are sterile on the inside and include an RTP integral to the container.

Turning now to FIGS. 6 and 7, and having particular reference to FIG. 6, before the carriers are introduced into the filling line, the poly bag over wrap is removed from each loaded carrier under unidirectional HEPA filtered air in order to maintain a low level of particulate matter and bio burden on the outside surface of the carriers. Following the poly bag over wrap removal, each carrier is individually tested for hermetic integrity by pressure decay, again under unidirectional HEPA filtered air.

Turning now to FIG. 7, in combination with FIG. 6, assuming that each carrier's integrity has been maintained throughout transport and E-beam sterilization, the carrier is introduced into the filling line, generally indicated at 170, by being passed through a UV decontamination tunnel 172, within which the outside of the carrier is surface decontaminated by UV radiation prior to the containers being removed from the carrier for filling. The carrier is inserted into the entry end of the UV tunnel 172, wherein UV emitting lights surround the carrier and irradiate the entire exterior surface to control potential contaminates from being introduced into subsequent isolators of the filling line.

After the UV cycle is complete, the carrier is transferred, through a chamber transfer door, into a carrier entry chamber (not shown) in which the carrier film lid is opened and the rail cartridge, including the containers, is removed from the carrier. Containers are removed from the rails of the cartridge and placed on tracks film which they are indexed onto a pick-and-place swing-arm 177 for transfer into a first filling isolator 174, which in the exemplary embodiment of the invention is a controlled environment for filling containers with, for example, powdered medicaments. Although the foregoing transfers may be accomplished by the use of automated equipment, transfer is typically performed manually, by reaching into the carrier entry station through "glove ports" or alternatively through the arums of a "half-suit" and manipulating the carrier, lid and cartridge. At this time, the empty rail cartridge and carrier are transferred back into the UV chamber and the transfer door is closed prior to removal of the cartridge and carrier from the UV chamber 172.

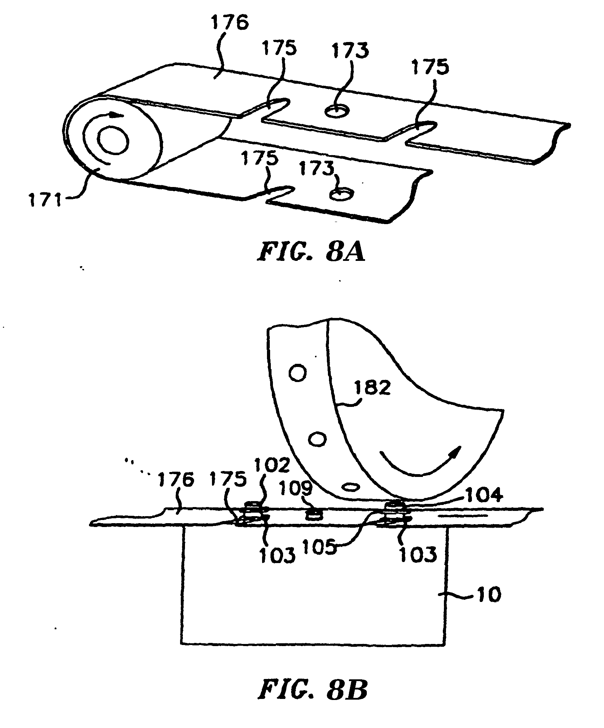

The pick-and-place arm 177 rotates a container to a position for loading onto a continuous-band transport mechanism, by means of which containers are introduced into the powder fill isolator 174 and subsequently indexed through the operational steps of the powder filling process. A portion of the continuous-band transport mechanism 176 is depicted in FIG. 8a and generally comprises a flat band of a suitable flexible material, such as metal or plastic and which forms a loop around the outside peripheral surface of a drive roller 171. The drive roller 171 is connected to a drive motor, such as a conventional D.C. motor or a stepper motor, which causes the transport band 176 to be indexed, in pre-determined, spaced-apart stages through the isolator. The transport band 176 includes a series of spaced-apart slots 175 which are cut into the band material in a direction orthogonal to the band's direction of travel. Each of the slots 175 has a width of about 12.4 mm, so as to accommodate the filling barrel of a container's sacrificial ports. Accordingly, the slots 175 are configured with a width sufficient to receive the filling barrel but are also sufficiently narrow to engage the underside surface of a sacrificial ports bottom most flange (103 of FIG. 1a).

A hole 173 is disposed intermediate a pair of slots, and is provided all the way through the material of the transport band 176. Each hole 173 has a diameter of about 11.0 mm and functions to provide a convenient receptacle for receiving a sacrificial port cap (109 of FIG. 1a) when a cap has been removed from a sacrificial port for filling Although the hole 173 is depicted in the exemplary embodiment of FIG. 8a as positioned equidistant between two slots 175, it will be evident that the location of the hole (or cap receptacle) 173 may be provided anywhere in proximity to the slots 175. If robotic pick-and-place equipment, for example, is used to remove the caps from the sacrificial ports, all that is required is that each cap receptacle 173 have a specific relationship to the slots 175 such that the position of the cap receptacle 173 may be programmed into the robotic equipment.

Returning now to FIG. 7, containers may be removed from the rail cartridge and loaded onto the transport band 176 by hand; an operator reaching into the isolator by means of a half-suit or flexible arm coverings, accessed through gasketed ports or, alternatively, containers can be loaded onto the transport band 176 by an automated pick-and-place swing-arm 177 which grasps each container and rotates it through approximately 90° to mate the sacrificial port flanges with the transport band recesses.

Initially, the transport band 176 indexes each container to a tare weight balance 178 (shown in FIG. 7) at which the tare weight of each container is determined in order to provide a reference empty weight against which to correlate subsequent checkweighs. The empty container may be removed from the transport band 176 and placed on the tare weight balance either by hand or by means of an automated pick-and-place robotic arm 179. Next, the container is reintroduced to the transport band and indexed to an aseptic, rotary, in-line powder feller 180. At the powder filler 180, a robotic arm 181 swings through an are to engage the cap on the sacrificial port of the medicament compartment (104 of FIG. 1). The cap is removed by grasping it by its removal flange (as described in connection with FIG. 1a.) and exerting a vertically upward force. After removal, the medicament compartment sacrificial port cap is deposited in the cap receptacle (173 of FIG. 8a) located between the sacrificial port slots on the transport band. The medicament compartment sacrificial port cap is now in a known location with respect to the medicament compartment sacrificial port, such that robotic machinery may now easily retrieve the cap for reinsertion into the medicament compartment sacrificial port as will be described further below. Following cap removal, the medicament compartment is opened (de-blocked) with a jet of 0.2 micron filtered nitrogen or air, introduced through the filling barrel of the medicament compartment sacrificial port.

Turning now to FIG. 8b, the transport band next indexes the container to a position where the medicament compartment sacrificial port is beneath a conventional, generally circular dosing wheel 182 which automatically dispenses a pre-determined amount of powdered medicament into the medicament compartment though the open port. The dosing wheel 182 is oriented in a direction orthogonal to the direction of travel of the transport band 176 and the containers 10 hung therefrom. Accordingly, the reason for the medicament compartment port's being taller than the diluent compartment's port now becomes apparent. In order that the entire powder charge contained in the dosing wheel it introduced into the medicament compartment without undue spillage, the medicament compartment's port is sized to place its open throat in proximity to the dosing wheel at a distance of approximately 1.0 millimeters. In order for the diluent compartment's port, including the still affixed cap, to clear the underside of the dosing wheel after dosing is completed and as the container is indexed to the next station, the total height of the diluent compartment port and cap combination must be no greater than the height of the medicament compartment port with the cap removed, i.e., no greater than the space between the transport band 176 and the dosing wheel 182.

An alternative configuration can be devised with regard to the orientation of the containers and the dosing wheel. For example, rather than traveling in-line, along their long axis, the containers could be introduced to the dosing wheel face-on, such that only the medicament compartment sacrificial port passes beneath the bottom of the arc of the dosing wheel. This particular orientation would allow the diluent port to avoid the narrow space between the transport band and the bottom of the dosing wheel are and thus preclude the necessity of providing sacrificial ports of separate heights.

Returning now to FIGS. 6 and 7, following powder fill, 0.2 micron filtered nitrogen gas or air is introduced into the medicament compartment's head space and the transport band 176 indexes the compartment to a heat seal station 184. Although both 0.2 micron filtered nitrogen gas or air are within the contemplation of the present invention, it will be understood that the choice between these two gasses, or other filter sterilized gasses (inert or otherwise) will depend upon the sensitivities of the particular drugs introduced into the medicament compartment. Specifically, if a drug is extremely sensitive to oxidation, the medicament compartment's head space will preferably be filled with filter sterilized nitrogen, or a similar inert gas. At the heat seal station 194 opposed heat seal heads are brought, together to either side of the container, to thereby close-off the channel (1 12b of FIG. 1) between the sacrificial port and the medicament compartment. The heat seal, thus formed, effectively continues permanent seals between the oversized edge seals 110e and 110f depicted in FIG. 1, thus sealing the medicament compartment.

Next the cap is reinserted into the medicament compartment sacrificial port and, the powder filled container is indexed to a gross weight balance 186 where its gross weight is taken to verify that the prosper amount of powdered drug has been dispensed into each container The gross weight, as determined at station 186 is conelated to the weight of the empty container as determined at the tare weight station 178. If the goss weight balance 186 determines that an improper amount of powdered drug has been introduced into the container's medicament compartment, the container is rejected and transferred to a reject rail cartridge for subsequent removal from the powder fill isolator 174. If the checkweigher determine that the amount of powdered drug in the medicament compartment is correct, the container is deemed to have been correctly filled and is indexed to the next station, or stations, if additional processing is desired.

In accordance with practice of principles of the invention, additional compartments of a container may be filled by additional medicaments or by diluents (follow-on filling) in a subsequent isolator unit, or multiple follow-on isolator units. Although the first filling step was described in connection with the introduction of a powdered drug into the medicament compartment, it will be understood that this was performed in the context of a multiple compartment medical container having separate compartments for a powdered medicament and a liquid diluent. However, it will be evident that a single compartment medical container can be filled in accordance with the present invention, by either a powdered drug, in the manner described above, or a liquid diluent or drug, in a manner to be described below.

Having particular reference to FIG. 7, the partially filled multiple compartment container of the exemplary embodiment of the invention, is now introduced to a second, liquid fill isolator unit 190 for aseptic filling with a diluent.

In particular, at the completion of the powder fill process, and as indicated in the exemplary process flow chart of FIG. 6, the partially filled container is moved from the powder fill isolator 174 to the liquid fill isolator 190 through a transfer tunnel 192 which is connected between the two isolator units. Following the above-described powder filling procedure, the container is removed from the transport band 176 of the powder fill isolator 174, and placed on a transfer band 194 which passes through the transfer tunnel 192 and which links the two isolator units. It will be understood by those having skill in the art that the transfer tunnel 192 and transfer band 194, in combination, provide an essential feature which promotes the modular nature of the isolator based filling process of the present invention. Indeed, it may be seen that a multiplicity of isolators may be linked together by transfer tunnels so as to add additional filling steps, with perhaps a multiplicity of ingredients, to the process. The modular nature of the container construction process, by which single or multiple compartment containers may be manufacture, interfaces easily with the modular nature of the filling process. As many fill isolators as are necessary to fill the desired number of compartments, may be easily linked together with transfer tunnels to thereby realize a manufacturing and filling of complete flexibility.

Returning now to the filling process and apparatus of FIGS. 6 and 7, the partially filled containers are introduced into the liquid fill isolator 190 through transfer tunnel 192 and are again placed on a continuous-loop transport band 196 which indexes the container through the steps of the liquid filling process.

As was the case with the previously described powder filling process, each container is indexed to a fill station 198 at which a robotic arm moves through an arc to grasp and remove the cap from the diluent compartment's sacrificial port and place it in a receptacle on the transport band. The diluent compartment is next de-blocked with a jet of 0.2 micron filtered nitrogen or air and advanced to place the diluent compartment sacrificial port beneath the dispensing nozzle of a diluent filling machine. A pre-determined amount of diluent, such as normal saline or 5% Dextrose Injection diluent is dispensed into the container through the sacrificial port. The diluent has typically been pre-mixed under qualified procedures in a separate mixing area and piped to the filling machine through 0.2 micron filters. It will be understood by those having skill in the art that diluent may be introduced to the container in a single dispensing step or, alternatively a dual dispensing step or multiple dispensing step procedure may be used in order to more accurately control the dose and minimize turbulence.

Following the diluent dispensing step the container is indexed to a diluent compartment heat seal station 200, where the diluent compartment head space is first adjusted with 0.2 micron filtered nitrogen or air. The heat seal station 200 comprises a heat seal platen opposed to a backing plate, which next are closed over the container so as to seal off the channel (112a of FIG. 1) between the diluent compartment and its sacrificial port. In effect, the diluent compartments heat seal continues the permanent peripheral seal between regions 110d and 110e of FIG. 1, thus completely sealing off the now completed container from the sacrificial strip.

The filled container now exits the liquid fill isolator 190 by an exit tunnel pass-through 202 and exit conveyor 204. The container is rinsed and dried to remove any residual diluent ared/or medicament from its exterior surface and is trimmed to its final dimensions by removing the oversized edge portion of the container which includes the sacrificial ports. As an optional part of the trimming process, the peripheral seal along the side of the container which is to be trimmed away may be reinforced thus ensuring the sepal of the medicament and diluent compartments on all sides of the container. Container fabrication and filling is now complete and the finished and filled container is folded-over along the seal between the medicament and diluent compartments, over-wrapped, and packed into shipping containers.

The production process for fabrication and filling of the container thus contemplates only a single sterilization procedure following the fabrication of the primary container. According to practice of principles of the invention, the construction of the container and the use of sacrificial ports communicating with the diluent and medicament compartments, allows the diluent and medicament compartments to be subsequently aseptically filled, and sealed without the need for any further sterilization procedures, Indeed, since the container of the present invention is not able to be terminally sterilized by steam, because of the high moisture sensitivity of powder medicaments and the moisture barrier properties of the medicament compartment cover, the aseptic filling methods, described above, are a necessary adjunct to the manufacture of a sterile final product. Fabrication and filling of the container in accordance with practice of the invention thus allows the container to be fabricated from materials, including high-barrier property laminates which are highly resistant to the effects of conventional steam sterilization. Once the requirement for down-stream sterilization is removed, medical containers may be constructed to include such high-barrier laminates, thus providing medical containers which are particularly suited for long term storage, and which may be efficiently fabricated with a low manufacturing cost.

In addition, it will be understood by those having skill in the art that the construction and use of the sacrificial ports communicating with the diluent and medicament compartments provides a means to index, retain, position and manipulate the container throughout the filling process. The size of the sacrificial port openings adapts the container to be compatible with conventional drug vial filling equipment technology.

The sacrificial ports are designed with flanges so that the container is able to be hung on an indexing mechanism and two flanges are provided on each port so that the container can be "handed off" by robotic pick-and-place equipment to off-line check weighing stations or be transferred between isolators. In addition, it will be seen that using sacrificial ports, in connection with medical container fabrication and filling processes, ideally promotes modularity in the fabrication and filling sequence.

## Claims

1. A transport carrier (150) adapted for sterilization by application of e-beam radiation, the transport carrier (150) comprising:
a generally rectangular container tray (152), the tray enclosing a volume and including an upper peripheral edge bent-over outwardly to form a horizontally oriented peripheral lip (156);
a rail cartridge (162) for supporting a multiplicity of containers (10), the rail cartridge (162) configured to be received within the volume of the tray (152); and
a sealable film lid (154) to be heat sealed to the horizontally oriented peripheral lip (156) to thereby cover the tray (152) to form a transportable sterile containment isolator for transporting and sterilizing empty containers.

2. The transport carrier (150) according to claim 1, wherein the sealable film lid (154) is constructed with dimensions along the film lid (154) to be positioned on the peripheral lip (156) such that the film lid edge is inset from an outer edge of the peripheral lip around the entire lip periphery.

3. The transport carrier (150) according to claim 1 or 2, wherein the sealable film lid (154) is heat sealed to the peripheral lip (156) of the tray (152).

4. The transport carrier (150) according to claim 3, wherein the film lid (154) heat seal extends beyond the edge of the film lid (154) and overlaps the film lid edge and the tray's peripheral lip (156), such that the entire periphery of the film lid (154) is heat sealed to the container's peripheral lip (156).

5. The transport carrier (150) according to any of claims 1 to 4, wherein the container tray (152) further comprises first and second opposing pockets (158, 160) formed in the centers of the opposing short sides of the tray (152) and extending outwardly from the plane of the short sides, the pockets (158, 160) extending downwardly from the peripheral lip (156) to form first and second opposing recesses for receiving the rail cartridge (162).

6. The transport carrier (150) according to any of claims 1 to 5, wherein the rail cartridge (162) includes means for engaging sacrificial ports (102, 104) of empty medical containers, such that a plurality of empty containers may be engaged and supported by the cartridge (162).

7. The transport carrier (150) according to any of claims 1 to 6, wherein the sacrificial port engagement means comprises a plurality of spaced-apart rails (163a, b, c, d, e, 1) defining slots (164a, b, c, d) therebetween, the sacrificial ports (102, 104) of medical containers inserted into the slots (164a-d), the slots including horizontally oriented flanges disposed along the length of each slot, the flanges engaging mating flanges on the sacrificial ports (102, 104) to support the ports thereby.

8. The transport carrier (150) according to any of claims 1 to 7, wherein the rail cartridge (162) further comprises a spacer plate (167), the spacer plate (167) affixed to the plurality of spaced-apart rails (163a-f), the spacer plate (167) including means for grasping and manipulating the rail cartridge (162) without contacting medical containers loaded thereon.

9. The transport carrier (150) according to any of claims 1 to 8, the rail cartridge (162) configured to be inserted into the container tray (152), the rail cartridge (162) having first and second ends adapted to be received into the container trays opposing pockets (158, 160).

10. A method for sterilizing a flexible container (10) adapted for administration of IV solutions, the flexible container (10) formed from a flexible front sheet (12) and a flexible rear sheet (14), the front and rear sheets (12, 14) sealed together at a common peripheral edge (16) so as to define a volume enclosure therebetween, at least one sacrificial port (102, 104) being interposed between the front and rear sheets (12, 14) and in communication with the volume enclosure, the front and rear sheets (12, 14) heated in a first localized area to fuse together the heated portions of the adjoining surfaces, thereby forming a permanent seal (110a, b, c) in a portion of the container extending between two sides of the common peripheral edge (16) and laterally spaced-away from the at least one sacrificial port (102, 104), the permanent seal (110a, b, c) defining a channel (112a, 112b) from the sacrificial port (102, 104) to the volume enclosure, the method comprising the steps of:
providing a transport carrier (150), the transport carrier (150) including a rail cartridge (162) configured to receive and support a multiplicity of containers (10) thereon, the rail cartridge (162) engaging the containers by their respective sacrificial ports (102, 104);
loading a multiplicity of containers (10) onto the rail cartridge (162);
placing the loaded rail cartridge (162) within a body portion of the transport carrier (150);
heat sealing the transport carrier (150) with a film lid (154) to thereby enclose the body portion; wrapping the sealed carrier in a poly bag and
exposing the sealed transport carrier (150) and containers within to an e-beam radiation source, the e-beam radiation source sterilizing the containers against biological contamination.

11. The method according to claim 10, further comprising:
introducing the e-beam sterilized sealed transport carrier (150) into a UV decontamination chamber;
decontaminating the exterior surfaces of the transport carrier (150) by application of UV radiation; and
introducing the decontaminated transport carrier (150) into an isolator (174, 190) having a sterile environment without reexposing the carrier (150) to ambient atmosphere.

12. The method according to claim 11, further comprising the steps of:
removing the sterilized containers from the transport carrier (150);
aseptically filling the volume enclosure through the sacrificial port (102, 104);
completing the permanent seal (110a, b, c) along the container's common peripheral edge (16) so as to interrupt communication between the sacrificial port (102, 104) and the volume enclosure; and
removing the sacrificial port (102, 104) from the container (10), whereby container formation is completed without being subject to a sterilization step after the volume enclosure is filled.

13. The method according to any of claims 10 to 12, wherein the volume enclosure is aseptically filled through the sacrificial port (102, 104) with a diluent liquid.

14. The method according to claim 12, wherein the volume enclosure is aseptically filled through the sacrificial port (102, 104) with a medicament.

15. The method according to any of claims 10 to 12, the transport carrier (150) further comprising:
a generally rectangular container tray (152) defining a body portion, enclosing a volume, the body portion including an upper peripheral edge, the edge bent-over outwardly to form a horizontally oriented peripheral lip (156); and
a flexible film (154) sealably affixed to the horizontally oriented peripheral lip (156) and extending over the body portion to thereby cover the container volume and form a transportable sterile containment isolator for transporting and sterilizing said containers (10).

16. The method according to claim 15, wherein the film (154) is sealably affixed onto the peripheral lip (156) such that a film peripheral edge in any direction is inset from a corresponding outer edge of the peripheral lip (156), around the entire lip periphery.

17. The method according to claim 16, wherein the film (154) is heat sealably affixed to the peripheral lip of the tray (152), the film heat seal extending beyond the edges of the film and overlapping the film edges and the carrier's peripheral lip (156) such that the entire peripheral edge of the film is heat sealed to the container's peripheral lip (156).

## Patentansprüche

1. Transportbehälter (150), der zur Sterilisierung durch Anwendung von Elektronenstrahlung eingerichtet ist, wobei der Transportbehälter (150) umfasst:
eine im Allgemeinen rechteckige Aufnahmewanne (152), wobei die Wanne ein Volumen umschließt und eine obere Umfangskante enthält, die nach außen umgebogen ist, so dass eine horizontal ausgerichtete Umfangslippe (156) entsteht;
einen Schieneneinsatz (162) zum Tragen einer Vielzahl von Behältnissen (10), wobei der Schieneneinsatz (162) so eingerichtet ist, dass er in dem Volumen der Wanne (152) aufgenommen wird; und
eine siegelbare Folienabdeckung (154), die an die horizontal ausgerichtete Umfangslippe (156) heißgesiegelt werden kann, um so die Wanne (152) abzudecken und einen transportfähigen sterilen Einschluss-Isolator zum Transportieren und Sterilisieren leerer Behältnisse zu schaffen.

2. Transportbehälter (150) nach Anspruch 1, wobei die siegelbare Folienabdeckung (154) mit Abmessungen entlang der Folienabdeckung (154) so aufgebaut ist, dass sie an der Umfangslippe (156) so positioniert wird, dass der Rand der Folienabdeckung um den gesamten Umfang der Lippe herum von einem äußeren Rand der Umfangslippe zurückgesetzt ist.

3. Transportbehälter (150) nach Anspruch 1 oder 2 wobei die siegelbare Folienabdeckung (154) an die Umfangslippe (156) der Wanne (152) heißgesiegelt ist.

4. Transportbehälter (150) nach Anspruch 3, wobei sich die Heißsiegelung der Folienabdeckung (154) über den Rand der Folienabdeckung (154) hinaus erstreckt und den Rand der Folienabdeckung sowie die Umfangslippe (156) der Wanne überlappt, so dass der gesamte Umfang der Folienabdeckung (154) an die Umfangslippe (156) des Behältnisses heißgesiegelt ist.

5. Transportbehälter (150) nach einem der Ansprüche 1 bis 4, wobei die Behälterwanne (152) des Weiteren eine erste und eine zweite Vertiefung (158, 160) umfasst, die einander gegenüberliegen, in der Mitte der einander gegenüberliegenden kurzen Seiten der Wanne (152) ausgebildet sind und sich von der Ebene der kurzen Seiten nach außen erstrecken, und sich die Vertiefungen (158, 160) von der Umfangslippe (156) nach unten erstrecken, so dass eine erste und eine zweite Aussparung zum Aufnehmen des Schieneneinsatzes (162) gebildet werden, die einander gegenüberliegen.

6. Transportbehälter (150) nach einem der Ansprüche 1 bis 5, wobei der Schieneneinsatz (162) eine Einrichtung enthält, die mit Einmal-Anschlüssen (102, 104) leerer medizinischer Behälter in Eingriff kommt, so dass die Vielzahl leerer Behälter mit dem Einsatz (162) in Eingriff kommen und von ihm getragen werden können.

7. Transportbehälter (150) nach einem der Ansprüche 1 bis 6, wobei die Einrichtung für Eingriff mit Einmal-Anschlüssen eine Vielzahl beabstandeter Schienen (163a, b, c, d, e, f) umfasst, zwischen denen Schlitze (164a, b, c, d) ausgebildet sind, die Einmal-Anschlüsse (102, 104) medizinischer Behälter in die Schlitze (164a-d) eingeführt werden, die Schlitze horizontal ausgerichtete Flansche enthalten, die in der Längsrichtung jedes Schlitzes angeordnet sind, und die Flansche mit passenden Flanschen an den Einmal-Anschlüssen (102, 104) in Eingriff kommen, um so die Anschlüsse zu tragen.

8. Transportbehälter (150) nach einem der Ansprüche 1 bis 7, wobei der Schieneneinsatz (162) des Weiteren eine Abstandshalteplatte (167) umfasst, die Abstandshalteplatte (167) an der Vielzahl beabstandeter Schienen (163a-f) befestigt ist und die Abstandshalteplatte (167) eine Einrichtung enthält, mit der der Schieneneinsatz (162) ergriffen und manipuliert wird, ohne mit den darauf aufgesetzten medizinischen Behältnissen in Kontakt zu kommen.

9. Transportbehälter (150) nach einem der Ansprüche 1 bis 8, wobei der Schieneneinsatz (162) so eingerichtet ist, dass er in die Aufnahmewanne (152) eingeführt wird, und der Schieneneinsatz (162) ein erstes sowie ein zweites Ende hat, die so eingerichtet sind, dass sie in den einander gegenüberliegenden Vertiefungen (158, 160) des Behälters aufgenommen werden.

10. Verfahren zum Sterilisieren eines flexiblen Behältnisses (10), der zur Verabreichung von I.V.-Lösungen eingerichtet ist, wobei das flexible Behältnis (10) aus einer flexiblen vorderen Bahn (12) und einer flexiblen hinteren Bahn (14) ausgebildet ist, die vordere und die hintere Bahn (12, 14) an einem gemeinsamen Umfangsrand (16) so aneinander gesiegelt sind, dass ein Einschlussvolumen dazwischen gebildet wird, wenigstens ein Einmal-Anschluss (102, 104) zwischen der vorderen und der hinteren Bahn (12, 14) angeordnet ist und mit dem Einschlussvolumen in Verbindung steht, die vordere sowie die hintere Bahn (12, 14) in einem ersten örtlich begrenzten Bereich erhitzt werden, um die erhitzten Abschnitte der aneinander grenzenden Flächen miteinander zu verschweißen und so eine dauerhafte Siegelung (110a, b, c) in einem Abschnitt des Behältnisses herzustellen, die sich zwischen zwei Seiten des gemeinsam Umfangsrandes (16) erstreckt und seitlich von dem wenigstens einen Einmal-Anschluss (102, 104) beabstandet ist, wobei die dauerhafte Siegelung (110a, b, c) einen Kanal (112a, 112b) von dem Einmal-Anschluss (102, 104) zu dem Volumeneinschluss bildet und das Verfahren die folgenden Schritte umfasst:
Bereitstellen eines Transportbehälters (150), wobei der Transportbehälter (150) einen Schieneneinsatz (162) enthält, der so eingerichtet ist, dass er eine Vielzahl von Behältnissen (10) aufnimmt und trägt, und der Schieneneinsatz (162) mit den Behältnissen über ihre jeweiligen Einmal-Anschlüsse (102, 104) in Eingriff ist;
Bestücken des Schieneneinsatzes (162) mit einer Vielzahl von Behältnissen (10);
Positionieren des bestückten Schieneneinsatzes (162) in einem Körperabschnitt des Transportbehälters (150);
Heißsiegeln des Transportbehälters (150) mit einer Folienabdeckung (154), um so den Körperabschnitt zu umschließen;
Umhüllen des gesiegelten Behälters mit einem Polybag; und
Einführen des gesiegelten Transportbehälters (150) und der Behältnisse in eine Elektronenstrahlungsquelle, wobei die Elektronenstrahlungsquelle die Behälter gegen biologische Kontamination sterilisiert.

11. Verfahren nach Anspruch 10, das des Weiteren umfasst:
Einleiten des mit Elektronenstrahlen sterilisierten gesiegelten Transportbehälters (150) in eine UV-Dekontaminationskammer;
Dekontaminieren der Außenflächen des Transportbehälters (150) durch Einwirkung von UV-Strahlung; und
Einleiten des dekontaminierten Transportbehälters (150) in einen Isolator (151) mit einer sterilen Umgebung, ohne den Behälter (150) erneut der Umgebungsatmosphäre auszusetzen.

12. Verfahren nach Anspruch 11, das des Weiteren die folgenden Schritte umfasst:
Entfernen der sterilisierten Behältnisse aus dem Transportbehälter (150);
aseptisches Füllen des Einschlussvolumens über den Einmal-Anschluss (102, 104);
Fertigstellen der permanenten Siegelung (110a, b, c) entlang des gemeinsamen Umfangsrandes (16) des Behälters, um Verbindung zwischen dem Einmal-Anschluss (102, 104) und dem Einschlussvolumen zu unterbrechen; und
Entfernen des Einmal-Anschlusses (102, 104) von dem Behälter, so dass Ausbildung des Behälters abgeschlossen wird, ohne dass er einem Sterilisierungsschritt unterzogen wird, nachdem das Einschlussvolumen gefüllt ist.

13. Verfahren nach einem der Ansprüche 10 bis 12, wobei das Einschlussvolumen über den Einmal-Anschluss (102, 104) aseptisch mit einer verdünnenden Flüssigkeit gefüllt wird.

14. Verfahren nach Anspruch 12, wobei das Einschlussvolumen über den Einmal-Anschluss (102, 104) aseptisch mit einem Medikament gefüllt wird.

15. Verfahren nach einem der Ansprüche 10 bis 12, wobei der Transportbehälter (150) des Weiteren umfasst:
eine im Allgemeinen rechteckige Aufnahmewanne (152), die einen Körperabschnitt aufweist, der ein Volumen umschließt, wobei der Körperabschnitt einen oberen Umfangsrand enthält und der Rand nach außen umgebogen ist, so dass eine horizontal ausgerichtete Umfangslippe (156) entsteht; und
eine flexible Folie (154), die siegelbar an der horizontal ausgerichteten Umfangslippe (156) befestigt wird und sich über den Körperabschnitt erstreckt, um so das Behältervolumen abzudecken und einen transportfähigen sterilen Einschluss-Isolator zum Transportieren und Sterilisieren der Behältnisse (10) zu bilden.

16. Verfahren nach Anspruch 15, wobei die Folie (154) siegelbar so an der Umfangslippe (156) befestigt wird, dass der Folien-Umfangsrand in jeder Richtung von einem entsprechenden äußeren Rand der Umfangslippe (156) um den gesamten Lippenumfang herum zurückgesetzt ist.

17. Verfahren nach Anspruch 16, wobei die Folie (154) heißsiegelbar an der Umfangslippe der Wand (152) befestigt wird und die Folien-Heißsiegelung über die Ränder der Folie hinaus ausgedehnt wird und die Folienränder und die Umfangslippe (156) des Behälter überlappt werden, so dass der gesamte Umfangsrand der Folie an die Umfangslippe (156) des Behältnisses heißgesiegelt wird.

## Revendications

1. Support de transport (150) pour effectuer une stérilisation par l'application d'un rayonnement de faisceau d'électrons, le support de transport (150) comprenant :
un bac récepteur (152) généralement rectangulaire, le bac entourant un volume et comportant un bord périphérique supérieur rabattu vers l'extérieur pour former une lèvre périphérique (156) orientée horizontalement ;
un chargeur à rail (162) servant à supporter plusieurs récipients (10), le chargeur à rail (162) étant conçu pour être reçu au sein du volume du bac (152) ; et
un couvercle de type film scellable (154) à thermo-sceller sur la lèvre périphérique (156) orientée horizontalement, pour ainsi recouvrir le bac (152) afin de former un confinement isolant stérile et transportable pour transporter et stériliser des récipients vides.

2. Support de transport (150) selon la revendication 1, dans lequel le couvercle de type film scellable (154) possède des dimensions telles que, lorsqu'il est positionné sur la lèvre périphérique (156), le bord du couvercle de type film est en retrait par rapport au bord externe de la lèvre périphérique autour de la totalité de la périphérie de lèvre.

3. Support de transport (150) selon la revendication 1 ou 2, dans lequel le couvercle de type film scellable (154) est thermo-scellé sur la lèvre périphérique (156) du bac (152).

4. Support de transport (150) selon la revendication 3, dans lequel le thermo-scellage du couvercle de type film (154) s'étend au-delà du bord du couvercle de type film (154) et recouvre le bord du couvercle de type film et la lèvre périphérique (156) du bac, de telle sorte que toute la périphérie du couvercle de type film (154) est thermo-scellée sur la lèvre périphérique (156) du récipient.

5. Support de transport (150) selon l'une quelconque des revendications 1 à 4, dans lequel le bac récepteur (152) comprend en outre une première poche et une seconde poche opposées (158, 160) formées au centre des petits côtés opposés du bac (152) et s'étendant vers l'extérieur à partir du plan des petits côtés, les poches (158, 160) s'étendant vers le bas à partir de la lèvre périphérique (156) pour former un premier renfoncement et un second renfoncement servant à recevoir le chargeur à rail (162).

6. Support de transport (150) selon l'une quelconque des revendications 1 à 5, dans lequel le chargeur à rail (162) comprend des moyens venant se loger dans les orifices sacrificiels (102, 104) de récipients médicaux vides, de sorte que plusieurs récipients vides peuvent être logés et supportés par le chargeur (162).

7. Support de transport (150) selon l'une quelconque des revendications 1 à 6, dans lequel les moyens venant se loger dans les orifices sacrificiels comprennent une pluralité de rails (163a, b, c, d, e, f) séparés les uns des autres entre lesquels des rainures (164a, b, c, d) sont définies, les orifices sacrificiels (102, 104) des récipients médicaux étant insérés dans les rainures (164a-d), les rainures comportant des brides orientées horizontalement disposées le long de la longueur de chaque rainure, les brides recevant des contre-brides se trouvant sur les orifices sacrificiels (102, 104) pour ainsi supporter les orifices.

8. Support de transport (150) selon l'une quelconque des revendications 1 à 7, dans lequel le chargeur à rail (162) comprend en outre une plaque d'entretoise (167), la plaque d'entretoise (167) étant fixée aux rails (163a-f) espacés les uns des autres, la plaque d'entretoise (167) comportant un moyen permettant de saisir et de manipuler le chargeur à rail (162) sans contact avec les récipients médicaux chargés sur celui-ci.

9. Support de transport (150) selon l'une quelconque des revendications 1 à 8, le chargeur à rail (162) étant conçu pour être inséré dans le bac récepteur (152), le chargeur à rail (162) possédant une première extrémité et une seconde extrémité pouvant être reçues dans les poches opposées (158, 160) du bac récepteur.

10. Procédé de stérilisation d'un récipient flexible (10) servant à administrer des solutions IV, le récipient flexible (10) étant formé à partir d'une plaque avant souple (12) et d'une plaque arrière souple (14), la plaque avant et la plaque arrière (12, 14) étant scellées ensemble le long d'un bord périphérique commun (16) afin de définir entre elles un volume clos, au moins un orifice sacrificiel (102, 104) étant intercalé entre la plaque avant et la plaque arrière (12, 14) et en communication avec le volume clos, la plaque avant et la plaque arrière (12, 14) étant chauffées dans une première zone localisée pour fusionner ensemble les parties chauffées des surfaces contiguës, pour ainsi former un joint permanent (110a, b, c) dans une partie du récipient se prolongeant entre deux côtés du bord périphérique commun (16) et séparée latéralement du au moins un orifice sacrificiel (102, 104), le joint permanent (110a, b, c) définissant un canal (112a, 112b) allant de l'orifice sacrificiel (102, 104) vers le volume clos, le procédé comprenant les étapes suivantes :
l'utilisation d'un support de transport (150), le support de transport (150) comportant un chargeur à rail (162) conçu pour recevoir et supporter plusieurs récipients (10), le chargeur à rail (162) se logeant dans les récipients par leurs orifices sacrificiels (102, 104) respectifs ;
le chargement de plusieurs récipients (10) sur le chargeur à rail (162) ;
le placement du chargeur à rail (162) chargé dans une partie corps du support de transport (150) ;
le thermo-scellage du support de transport (150) avec un couvercle de type film (154) pour ainsi fermer la partie corps ;
l'emballage du support scellé dans une enveloppe en polyéthylène ; et
l'exposition du support de transport (150) scellé et des récipients qu'il contient à une source de rayonnement de faisceau d'électrons, la source de rayonnement de faisceau d'électrons stérilisant les récipients par destruction des contaminants biologiques.

11. Procédé selon la revendication 10, comprenant en outre :
l'introduction du support de transport (150) scellé et stérilisé sous un faisceau d'électrons dans une chambre de décontamination sous UV ;
la décontamination des surfaces externes du support de transport (150) par l'application d'un rayonnement UV ; et
l'introduction du support de transport (150) décontaminé dans un isolateur (174, 190) comportant un environnement stérile sans exposer à nouveau le support (150) à l'atmosphère ambiante.

12. Procédé selon la revendication 11, comprenant en outre les étapes suivantes :
le retrait des récipients stérilisés du support de transport (150) ;
le remplissage en condition aseptique du volume clos par l'intermédiaire de l'orifice sacrificiel (102, 104) ;
l'achèvement de la formation du joint permanent (110a, b, c) le long du bord périphérique commun (16) du récipient afin d'interrompre la communication entre l'orifice sacrificiel (102, 104) et le volume clos ; et
le retrait de l'orifice sacrificiel (102, 104) du récipient (10), moyennant quoi la formation du récipient est terminée sans qu'il ait été soumis à une étape de stérilisation après le remplissage du volume clos.

13. Procédé selon l'une quelconque des revendications 10 à 12, dans lequel le volume clos est rempli en condition aseptique par l'intermédiaire de l'orifice sacrificiel (102, 104) avec un diluant liquide.

14. Procédé selon la revendication 12, dans lequel le volume clos est rempli en condition aseptique par l'intermédiaire de l'orifice sacrificiel (102, 104) avec un médicament.

15. Procédé selon l'une quelconque des revendications 10 à 12, le support de transport (150) comprenant en outre :
un bac récepteur (152) généralement rectangulaire définissant une partie corps, enfermant un volume, la partie corps comportant un bord périphérique supérieur, le bord étant rabattu vers l'extérieur pour former une lèvre périphérique (156) orientée horizontalement ; et
un film flexible (154) fixé par scellement à la lèvre périphérique (156) orientée horizontalement et se prolongeant sur la partie corps pour ainsi recouvrir le volume du récipient et former un confinement isolant stérile et transportable servant à transporter et à stériliser lesdits récipients (10).

16. Procédé selon la revendication 15, dans lequel le film (154) est fixé par scellement sur la lèvre périphérique (156), de telle sorte qu'un bord périphérique du film est en retrait dans n'importe quelle direction par rapport à un bord externe correspondant de la lèvre périphérique (156) autour de la totalité de la périphérie de la lèvre.

17. Procédé selon la revendication 16, dans lequel le film (154) est fixé par thermo-scellage sur la lèvre périphérique du bac (152), le thermo-scellage du film s'étendant au-delà des bords du film et recouvrant les bords du film et la lèvre périphérique (156) du support, de telle sorte que tout le bord périphérique du film est thermo-scellé sur la lèvre périphérique (156) du récipient.
